# EUROPEAN PATENT APPLICATION

(11) **EP 1 900 432 A2**
(43) Date of publication of application: **19.03.2008**
(21) Application number: 07109720.8
(22) Date of filing: 06.06.2007
(51) Int. Cl.: B01L 3/00, G01N 33/543

(54) **Magnetic bead-packing unit using centrifugal force, microfluidic device including the same, and immunoassay method using the microfluidic device**

(30) Priority: 30.08.2006 KR 20060082941
(71) Applicant: Samsung Electronics Co., Ltd., Suwon-si, Gyeonggi-Do (KR)
(72) Inventor: Cho, Yoon-kyoung, Yongin-si Gyeonggi-do (KR); Lee, Jeong-gun, Yongin-si Gyeonggi-do (KR); Lee, Beom-seok, Yongin-si Gyeonggi-do (KR)
(74) Representative: Grünecker, Kinkeldey, Stockmair & Schwanhäusser Anwaltssozietät

(57) **Abstract**

A magnetic bead-packing unit using centrifugal force, a microfluidic device including the same, and an immunoassay method using the microfluidic device are provided. The magnetic bead-packing unit includes a rotary body controllably rotating; a microfluidic channel disposed on the rotary body and including an inlet and an outlet disposed farther from a rotation center of the rotary body than the inlet to provide a channel through which fluid flows from the inlet to the outlet due to centrifugal force, wherein the microfluidic channel comprises a curved portion in which the microfluidic channel first extends away from the rotation center of the rotary body and then turns toward the rotation center of the rotary body; and a magnet disposed to correspond to the curved portion and applying a magnetic force to fluid flowing through the microfluidic channel. The immunoassay method includes preparing a sample solution mixed with magnetic beads in the sample solution chamber; and packing the magnetic beads in the curved portion by passing the sample solution mixed with the magnetic bead through the microfluidic channel due to centrifugal force caused by rotation of the rotary body.

## Description

### BACKGROUND OF THE INVENTION

### 1. Field of the Invention

The present invention relates to a magnetic bead-packing unit using centrifugal force, a microfluidic device including the same, and an immunoassay method using the microfluidic device, and more particularly, to a microfluidic device capable of effectively packing magnetic beads and an immunoassay method using the microfluidic device.

### 2. Description of the Related Art

An immunoassay is a test comprising marking any one or both of an antigen and an antibody with a radioisotope, a chemiluminescent material, or the like and measuring the existence of the antigen or the antibody through an antigen-antibody reaction. A method using the radioisotope is known as a radioimmunoassay, and a method using the chemiluminescent material is known as an immunofluorescence.

Recently, research has been conducted into ways of performing such an immunoassay using a biochip, a biosensor, a microfluidic device, or the like, fabricated using a minute fabrication technology. In order to perform an immunoassay based on a solid surface using microbeads in a microfluidic device, it is necessary to pack the beads in a predetermined space such as a channel or a chamber through which a sample solution flows. Therefore, a structure is needed to contain a number of beads in the microfluidic device. Recently, research has been conducted into ways of performing various operations while moving fluid in a compact disc (CD)-shaped microfluidic device using centrifugal force. A device capable of effectively packing beads using centrifugal force is needed in order to perform an immunoassay based on a solid surface using beads in such a CD-shaped microfluidic device.

When beads are packed in a microfluidic device, if the diameter of the beads is too small, as are commercially used magnetic beads, it is difficult to fabricate a structure to contain the beads. In addition, fluid pressure in the microfluidic device is greatly reduced as the fluid passes through the packed beads. In particular, in a CD-shaped microfluidic device, the pressure loss results in having to increase the rotation speed of the device in order to transfer the fluid. On the other hand, if the diameter of the beads is too large, the pressure loss can be reduced. However, in this case, the gap between the packed beads is increased, lowering the efficiency of an immune reaction.

### SUMMARY OF THE INVENTION

The present invention provides a magnetic bead-packing unit and a microfluidic device having the same capable of effectively packing magnetic beads using centrifugal force and magnetic force in a microfluidic structure disposed on a rotary body.

The present invention also provides a method of effectively performing an immunoassay using the microfluidic device and a magnetic bead having a size adequate for packing.

According to an aspect of the present invention, there is provided a magnetic bead-packing unit including: a rotary body controllably rotating; a microfluidic channel disposed on the rotary body and including an inlet and an outlet disposed farther away from a rotation center of the rotary body than the inlet to provide a flow passage through which fluid flows from the inlet to the outlet due to centrifugal force, wherein the microfluidic channel includes a curved portion in which the flow passage extends away from the rotation center of the rotary body and then turns toward the rotation center of the rotary body; and a magnet disposed to correspond to the curved portion and applying a magnetic force to fluid flowing through the microfluidic channel.

The microfluidic channel may further comprise a bottleneck portion in which the flow passage is reduced in size at a position close to the curved portion and the bottleneck portion may be disposed at a rear end portion of the curved portion along the flow passage. The magnet may be fixed to the rotary body at a position adjacent to the curved portion or be formed in the shape of a ring having a radius corresponding to the distance from the rotation center of the rotary body to the curved portion, and is disposed outside the rotary body. The curved portion may be U-shaped or V-shaped such that an outer portion of the curved portion faces a rim of the rotating body. The rotary body may have various shapes adequate for rotating, for example, be a compact disc-shaped rotating plate.

According to another aspect of the present invention, there is provided a microfluidic device including: a rotary body controllably rotating and including a sample solution chamber, and a waste chamber disposed farther from a rotation center of the rotary body than the sample solution chamber; a microfluidic channel disposed on the rotary body and including an inlet connected with the sample solution chamber and an outlet connected with the waste chamber to provide a flow passage through which fluid flows from the inlet to the outlet due to centrifugal force, wherein the microfluidic channel includes a curved portion in which the flow passage first extends from the rotation center of the rotary body and then turns the rotation center of the body; and a magnet disposed to correspond to the curved portion and applying a magnetic force to fluid flowing through the microfluidic channel.

The microfluidic channel may further comprise a bottleneck portion in which the flow passage is reduced in size at a position close to the curved portion and the bottleneck portion may be disposed at a rear end portion of the curved portion along the flow passage. The magnet may be fixed to the rotary body at a position adjacent to the curved portion or be formed in the shape of a ring having a radius corresponding to the distance from the rotation center of the rotary body to the curved portion outside the rotary body. The curved portion may be U-shaped or V-shaped such that an outer portion of the curved portion faces a rim of the rotating body. The rotary body may have various shapes adequate for rotating, for example, be a compact disc-shaped rotating plate.

According to another aspect of the present invention, there is provided an immunoassay method using the microfluidic device. The immunoassay method according to present invention includes: preparing a sample solution mixed with magnetic beads in the sample solution chamber; and packing the magnetic beads in the curved portion by passing the sample solution mixed with the magnetic beads through the microfluidic channel due to centrifugal force caused by a rotation of the rotary body.

The diameter of the magnetic bead may range from 10 to 50 µm. Each of the magnetic beads may include a core formed of a magnetic material and a shell formed of a nonmagnetic material to surround the core. The shell may be formed of a biologically inert polymer material and a surface of the shell may be biologically activated.

In more detail, the shell may be formed of any one material selected from the group consisting of styrene, agarose, dextran, and polyethylene glycol (PEG). The surface of the shell may be modified such that predetermined biomolecules are specifically bound thereto. The surface of the shell may have at least one probe selected from the group consisting of an antibody, antigen, DNA, biotin, protein, amino group (NH₂-), and carboxyl group (COOH-). The surface of the shell may be formed of silica.

### BRIEF DESCRIPTION OF THE DRAWINGS

The above and other features and advantages of the present invention will become more apparent by describing in detail exemplary embodiments thereof with reference to the attached drawings in which:
FIGS. 1A and 1 B are plan views of a microfluidic device before and after packing magnetic beads, respectively, according to an embodiment of the present invention;
FIG. 2 is a three-dimensional image of a structure illustrated in FIGS. 1A and 1B, according to an embodiment of the present invention;
FIG. 3 is a sectional view taken along a line III-III of FIG. 2, according to an embodiment of the present invention;
FIGS. 4 and 5 are microphotographic images of magnetic beads used in performing an immunoassay using a microfluidic device according to an embodiment of the present invention;
FIG. 6 is a sequence of photographic images illustrating a procedure of packing magnetic beads using a microfluidic device in temporal order according to an embodiment of the present invention; and
FIG. 7 is a sequence of photographic images illustrating a procedure of performing an immunoassay using a microfluidic device in temporal order according to an embodiment of the present invention.

### DETAILED DESCRIPTION OF THE INVENTION

Hereinafter, the present invention will now be described in detain with reference to the accompanying drawings.

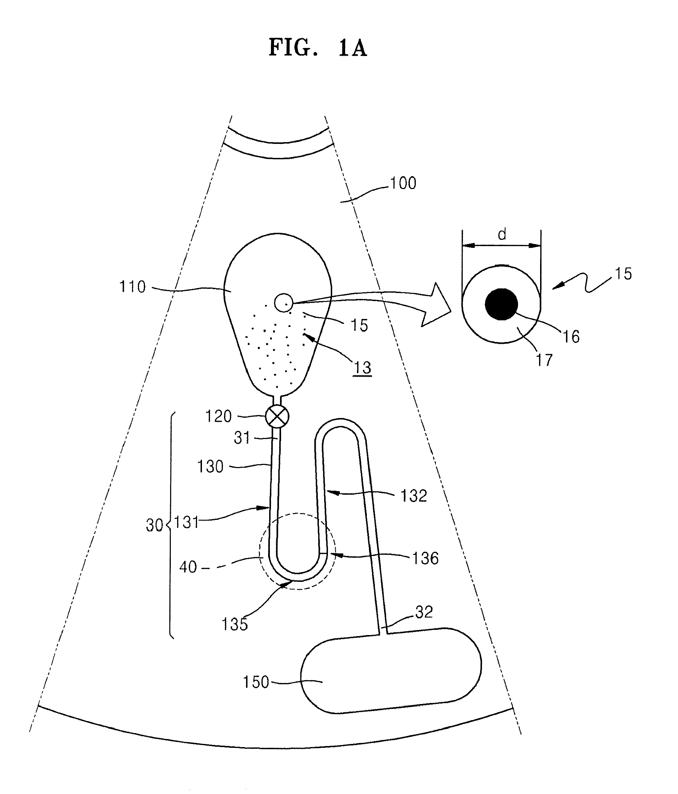

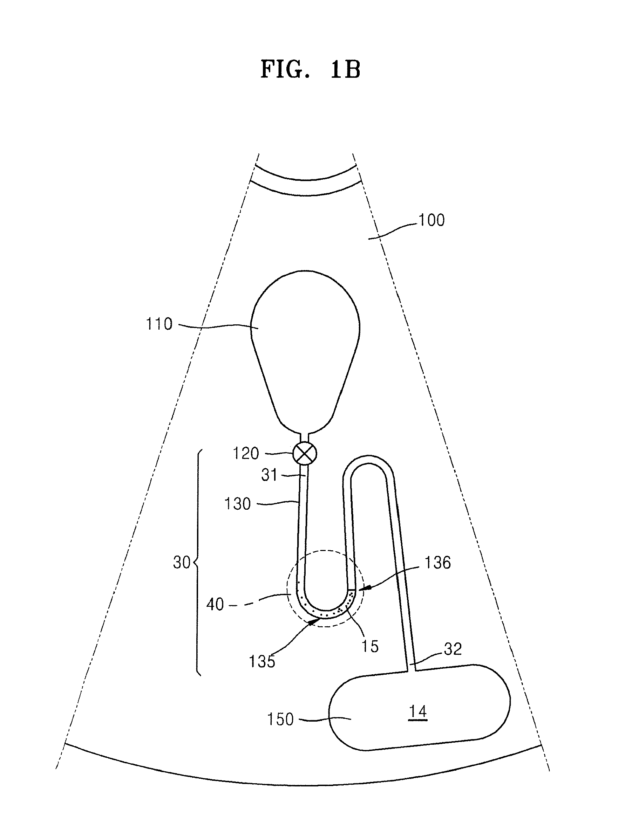

FIGS. 1A and 1B are plan views of a microfluidic device before and after packing magnetic beads ,respectively, according to an embodiment of the present invention.

Referring to FIGS. 1A and 1B, the microfluidic device includes a rotating plate 100. A magnetic bead-packing unit 30 according to an embodiment of the present invention is disposed on the rotating plate 100. The rotating plate 100 is an example of a rotary body that controllably rotates, but the present invention is not limited thereto. The microfluidic device also includes a microfluidic channel 130 that includes an inlet 31 and an outlet 32 and provides a channel through which fluid flows due to a pressure difference between the inlet 31 and the outlet 32. The inlet 31 is disposed close to the rotation center of the rotating plate 100 and the outlet 32 is disposed farther from the rotation center of the rotating plate 100 than the inlet 31. Therefore, a pressure difference between the inlet 31 and the outlet 32 is generated by centrifugal force due to the rotation of the rotating plate 100.

The microfluidic channel 130 includes a U-shaped or V-shaped curved portion 135, in which a fluid passage extends away from the rotation center of the rotating plate 100 and then turns back toward the rotation center of the rotating plate 100. The microfluidic channel 130 may further include a bottleneck portion 136 in which a section of the microfluidic channel 130 suddenly becomes smaller in size/ at a position close to the curved portion 135. The bottleneck portion 136 allows magnetic beads 15 to be contained in the curved portion 135 by slackening a flow of a sample solution 13 mixed with the magnetic beads 15.

A magnet 40 is disposed at a position adjacent to the curved portion 135. The magnet 40 may be disposed in front of the bottleneck portion 136 which is disposed at the rear of the curved portion 135. The magnet 40 may apply a magnetic force to pack the magnetic beads 15 mixed in the sample solution 13 in the curved portion 135.

The microfluidic device according to the current embodiment of the present invention includes a sample solution chamber 110 and a waste chamber 150 disposed on the rotating plate 100. The sample solution chamber 110 is disposed closer to the rotation center of the rotating plate 100 than the waste chamber 150. The microfluidic channel 130 is disposed between the sample solution chamber 110 and the waste chamber 150 to connect the sample solution chamber 110 and the waste chamber 150. The curved portion 135 is disposed such that an outer portion of the curved portion 135 faces away from the rotation center of the rotating plate 100, that is, towards a rim of the rotating plate 100. The curved portion 135 is disposed between an inlet channel 131 of the inlet 31 connected with the sample solution chamber 110 and an outlet channel 132 of the outlet 32 connected with the waste chamber 150. The bottleneck portion 136 in which a section of the fluid passage becomes suddenly reduced in size may be disposed in an end of the curved portion 135.

The magnet 40 may be disposed on or under or both on and under the curved portion 135. When the magnet 40 is disposed under the microfluidic channel 130, it is easy to observe a surface of the magnetic beads 15 packed in the microfluidic channel 130. The magnet 40 may be a permanent magnet or an electromagnet. In particular, a permanent magnet has a high magnetic flux density and a small size, thus having excellent portability. Meanwhile, the magnet 40 may be ring-shaped, wherein the radius of rotation of the magnet is the distance from the rotation center of the rotating plate 100 to the curved portion 135, and be disposed outside the rotating plate 100 to correspond to a trace of a rotation of the curved portion 135 (not shown).

A valve 120 may be disposed between the inlet 31 of the microfluidic channel 130 and the sample solution chamber 110 to control a flow of fluid. The valve 120 may be, for example, a capillary valve or a hydrophobic valve that is opened and closed by controlling the amount of rotation of the rotating plate 100.

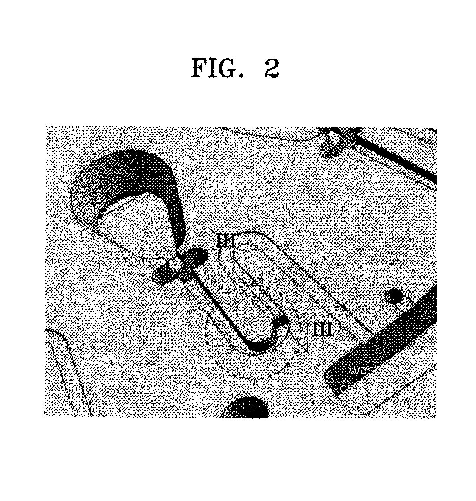

An operation of the microfluidic device illustrated in FIGS. 1 and 2 according to an embodiment of the present invention will now be described. The sample solution 13 mixed with the magnetic beads 15 is injected into the sample solution chamber 110. When the valve 120 is opened and the rotating plate 100 rotates, the pressure increases around the inlet 31 of the microfluidic channel 130 in which fluid is collected by a centrifugal force and the pressure decreases around the outlet 32 of the microfluidic channel 130 through which fluid flows out by a centrifugal force, which allows the sample solution 13 to flow in the microfluidic channel 130.

When the sample solution 13 mixed with the magnetic beads 15 reaches the curved portion 135 disposed at an entrance of the bottleneck portion 136, the speed of the magnetic beads 15 is reduced by the bottleneck portion 136 and the magnetic beads 15 may be packed in the curved portion 135 due to the magnetic force of the magnet 40 (refer to FIG. 1 B). Also, since the curved portion 135 is curved toward the rim of the rotating plate 100, the magnetic beads 15 cannot pass through the bottleneck portion 136. The magnetic beads 15 have generally a higher density than the sample solution 13 and are affected by a centrifugal force more than the sample solution 13, and thus hardly move back towards the rotation center of the rotating plate 100. Thus, the magnetic beads 15 are separated from the sample solution 13, and a remaining waste 14 is discharged to the waste chamber 150 through the outlet channel 132.

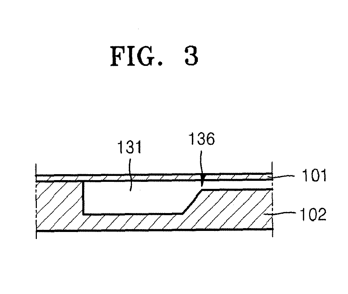

FIG. 2 is a three-dimensional image of the microfluidic illustrated in FIGS. 1A and 1B, according to an embodiment of the present invention. Intaglio structures composing the sample solution chamber 110, the microfluidic channel 130, and the waste chamber 150 are formed in a lower section of the rotating plate 100. A transparent cover (not shown) may be disposed thereon. FIG. 3 is a sectional view taken along a line III-III of FIG. 2, according to an embodiment of the present invention. A lower section 102 of the rotating plate, a cover 101, and the inlet channel 131 and the bottleneck portion 136 formed in the lower plate 102 are illustrated in FIG. 3. However, the present invention may be embodied in various shapes, for example, the microfluidic device as above may be disposed in the cover 101.

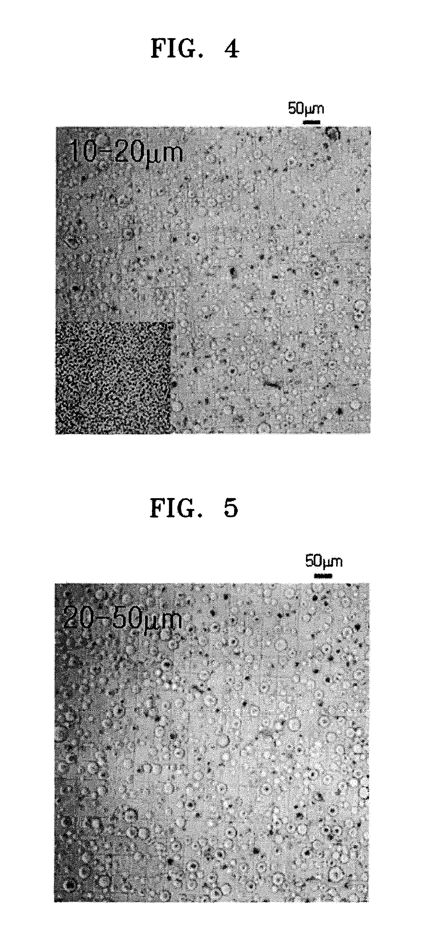

FIGS. 4 and 5 are microphotographic images of magnetic beads used in performing an immunoassay using a microfluidic device according to an embodiment of the present invention. FIG. 4 illustrates magnetic beads having a diameter of approximately 10 to 20 µm and FIG. 5 illustrates magnetic beads having a diameter of approximately 20 to 50 µm. Each of the magnetic beads 15 (refer to an enlarged view illustrated in FIG. 1A) includes a core 16 that is a magnetic particle and a shell 17 that is a coating layer surrounding the core 16. A diameter d of the magnetic beads 15 may range from 10 to 50 µm.

The magnetic beads 15 are relatively large-sized, compared to commercially used magnetic beads having a diameter less than 10 µm. If the diameter of the magnetic beads 15 is too small, the sample solution 13 flowing through the packed magnetic beads 15 experiences a significant pressure drop. Also, the operating efficiency decreases in a process for a surface treatment of the packed magnetic beads 15, for example, washing, attachment of biochemical substrates, or the like. On the other hand, if the diameter of the magnetic beads 15 is too large, since the porosity of the packed magnetic beads 15 increases, a large amount of biomolecules to be included and detected in the sample solution 13 pass through the magnetic beads 15 without contacting a surface of the magnetic beads 15, and thus are not suitable for performing an immunoassay.

The core 16 of the magnetic beads 15 may be any particle having magnetism and having a diameter of several tens of nm to several µm similar to general magnetic beads. In particular, the core 16 may include at least one material selected from the group of ferromagnetic metals consisting of Fe, Ni, Cr and oxides thereof.

The shell 17 of the magnetic beads 15 may be formed of a nonmagnetic material that does not shield a magnetic field. Also, since the shell 17 has many chances to contact a biological sample, the shell 17 may be biologically inert to prevent an unnecessary biochemical reaction. The shell 17 may include styrene, agarose, dextran, polyethylene glycol (PEG), etc. as a material that satisfies such a condition.

The surface of the shell 17 may be biologically activated for nonspecific or specific binding with biomolecules. The biological activation includes a chemical surface modification. For example, the surface of the shell 17 may be modified with silica such that a nucleic acid is nonspecifically bound to the surface of the shell 17 of the magnetic beads 15. In this case, a nucleic acid may be extracted or refined by binding a nucleic acid to the surface of the magnetic beads 15 from a nucleic acid extraction solution including a cell solution mixed with chaotropic salt, NaOH solution, etc.

The surface of the shell 17 may be modified such that a predetermined molecule can be specifically attached thereto. The shell 17 may include at least one probe selected from the group consisting of an antibody, antigen, DNA, biotin, protein, amino group (NH₂-), and carboxyl group (COOH-) that can be specifically bound with a predetermined molecule to be detected in the surface of the shell 17. Since the shell 17 is formed of a biologically inert material, the surface of the shell 17 may be previously coated with a material such as an epoxy, streptavidine, etc. to form the probe. When the magnetic beads 15 are surface-modified with an antibody (that is, have probes formed of an antibody), the magnetic beads 15 are useful for detecting an antigen of low concentration from a small amount of a sample, because an antibody can selectively capture a specific antigen.

Hereinafter, an exemplary method of fabricating magnetic beads to be used for a microfluidic device for an immunoassay according to an embodiment of the present invention will be briefly described.
- fabrication of magnetic nanoparticles
   A 20 ml solution of 0.4 M FeCl₂ and 0.25 M FeCl₃ was prepared, and then mixed by stirring for 30 minutes at a room temperature. 20 ml of 5 M NaOH solution was slowly added to the aforementioned solution and stirred for 10 minutes to form magnetic particles. The magnetic particles were washed by distilled water until they were neutralized. The magnetic particles were thoroughly dried at 80°C and then a dry weight of the magnetic particles was determined.
- fabrication of magnetic agarose beads
   4 g of Agarose was added to 100 ml of distilled water and then melted by a microwave oven. 4 g of magnetic particles were added to the distilled water and then stirred well. 5 g of Ethyl cellulose was melted in 100 ml of toluene and then stirred and thoroughly melted for 2 hours or more at 60°C. The aforementioned agarose solution was slowly added to the ethyl cellulose solution and then stirred for 30 minutes. The resulting solution is cooled down to a temperature of 25°C, the resulting solution was washed with 100% ethyl alcohol twice, 70% ethyl alcohol twice, and with distilled water several times.
   The generated beads were observed using a microscope. The agarose beads fabricated using the aforementioned method had a diameter of approximately 10 to 50 µm.
- cross-linking and activation of magnetic agarose beads
   100 ml of Agarose beads were added to 100 ml of 0.5 N sodium hydroxide, 10 ml of 1, 4-butanediol diglycidyl ether or epichlorohydrin were added thereto and then stirred and reacted for 8 hours at 25°C. The resulting solution was washed with 5 L of distilled water, the number of epoxy group molecules introduced in the beads was measured using HCl titration of epoxy molecules dissociated and generated in 3M Na₂S₂O₃ solution, and kept at 4°C.
- conjugation of an antibody
   A method of conjugating an antibody to the epoxy-activated magnetic agarose beads fabricated using the aforementioned method will be described. An antibody diluted at a concentration of 0.1 to 1 mg/ml (50 mM sodium carbonate buffer) was mixed in beads washed by 50 mM sodium carbonate buffer (pH 9.0) several times and reacted for 16 hours or more at 4°C. The amount of the antibody remaining in the reaction solution was measured by determining the amount of a protein using a Bradford method and converted to measure an amount of the antibody conjugated to the beads. The reacted beads were washed with 50 mM sodium carbonate buffer several times and then processed with 1 M glycine solution for 12 hours to remove the active group without the antibody attached. The beads were neutralized by washing by PBS several times and kept at 4°C. The activation of the antibody conjugated to the beads was observed by an ELISA method using a plate coated with an antigen.

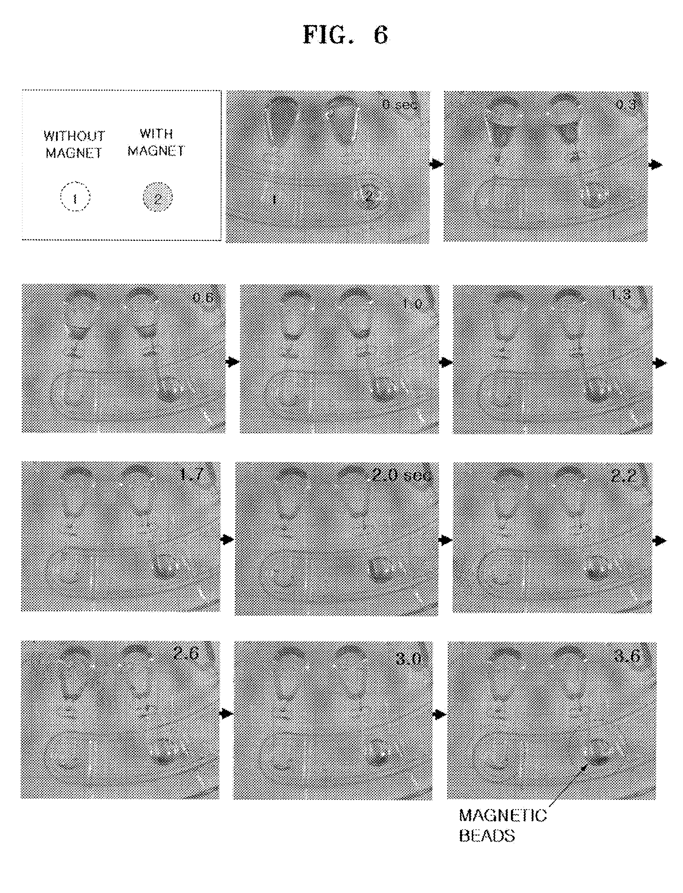

FIG. 6 is a sequence of photographic images illustrating a procedure of packing magnetic beads using a microfluidic device in temporal order according to an embodiment of the present invention. A microfluidic device having a magnetic bead-packing unit according to an embodiment of the present invention is illustrated on the right side of each of the photographic images and a microfluidic device without a magnet is illustrated on the left side for comparing to the present invention. The number illustrated in a right upper portion of each of the photographic images represents time elapsed in units of seconds.

The prepared magnetic beads 15 and sample solution 13 are injected into the sample solution chamber 110 and mixed. While the rotating plate 100 of the CD-shaped microfluidic device is rotated, the valve 120 connected with the outlet of the sample solution chamber 110 is opened. When the sample solution 13 mixed with the magnetic beads 15 passing through the microfluidic channel 130 reaches the curved portion 135, the magnetic beads 15 are packed in the curved portion 135 due to magnetic force and centrifugal force, and the sample solution waste moves to the waste chamber 150. After 3 seconds, it can be found that the magnetic beads 15 are packed in the curved portion 135 adjacent to the magnet 40.

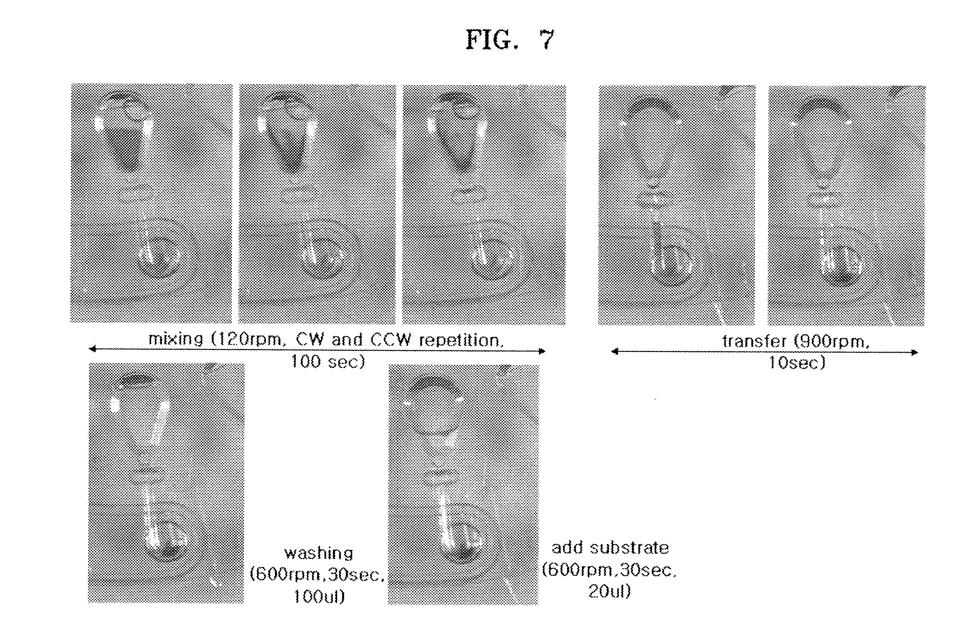

FIG. 7 is a sequence of photographic images illustrating a procedure of performing an immunoassay using a CD-shaped microfluidic device in temporal order according to an embodiment of the present invention. The sample solution 13 including an antigen is injected into the sample solution chamber 110, and the prepared magnetic beads 15 are mixed therein. The mixing operation can be performed by repeatedly rotating the rotating plate 100 of the microfluidic device in both directions. In the present embodiment, the rotating plate100 is repeatedly rotated at 120 rpm for 100 seconds in clockwise and counterclockwise directions. Meanwhile, the magnetic beads 15 previously mixed in the sample solution 13 may be injected into the sample solution chamber 110.

Next, the sample solution 13 mixed with the magnetic beads 15 moves through the microfluidic channel due 130 to centrifugal force. In the present embodiment, the rotating plate 100 is rotated at 900 rpm for 10 seconds in any direction. As illustrated in FIG. 7, the sample solution 13 mixed with the magnetic beads 15 moves through the microfluidic channel 130 and the magnetic beads 15are packed in the curved portion 135.

Next, the buffer solution is injected into the sample solution chamber 110 and the packed magnetic beads 15 can be washed while moving the buffer solution due to centrifugal force. In the present embodiment, while rotating the rotating plate 100 at 600 rpm, the packed magnetic beads 15 are washed using 100 µl of the buffer solution for 30 seconds. Next, 20 µl of biochemical substrate for detecting an immune reaction may be injected separately or simultaneously in the same way as the washing operation to attach the biochemical substrate to a surface of the packed magnetic beads 15. The immune reaction may be detected using a method of measuring a fluorescence, a color, or a radioisotope according to a kind of the biochemical substrate. The immune reaction may be quantitatively determined.

According to the present invention, magnetic beads can be effectively packed using a centrifugal force and a magnetic force in a microfluidic structure disposed in a rotary body. That is, a magnetic bead-packing unit and a microfluidic device including the same have simplified structures and thus the microfluidic device can be miniaturized, can reduce driving power, and can derive a high efficiency immune reaction.

Also, since the immunoassay method according to the present invention uses the microfluidic device and adequately-sized magnetic beads according to the present invention, the immunoassay method can be conveniently performed with low driving energy.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A magnetic bead-packing unit comprising:
a rotary body (100) controllably rotating;
a microfluidic channel (130,131,132,135,136) disposed on the rotary body and comprising an inlet (31) and an outlet (32) disposed farther away from a rotation center of the rotary body than the inlet to provide a flow passage through which fluid flows from the inlet to the outlet due to centrifugal force, wherein the microfluidic channel comprises a curved portion (135) in which the flow passage first extends away from the rotation center of the rotary body and then turns toward the rotation center of the rotary body; and
a magnet (40) disposed to correspond to the curved portion (135) and applying a magnetic force to fluid flowing through the microfluidic channel.

2. The magnetic bead-packing unit of claim 1, wherein the microfluidic channel further comprises a bottleneck portion (136) in which the flow passage is reduced in size at a position close to the curved portion.

3. The magnetic bead-packing unit of claim 2, wherein the bottleneck portion (136) is disposed at a rear end portion of the curved portion (135) along the flow passage.

4. The magnetic bead-packing unit according to one of the previous claims, wherein the magnet (40) is fixed to the rotary body (100) at a position adjacent to the curved portion (135).

5. The magnetic bead-packing unit according to one of the previous claims, wherein the magnet (40) is formed in the shape of a ring having a radius corresponding to the distance from the rotation center of the rotary body to the curved portion (135), and is disposed outside the rotary body.

6. The magnetic bead-packing unit according to one of the previous claims, wherein the curved portion (135) is U-shaped or V-shaped such that an outer portion of the curved portion faces a rim of the rotating body.

7. The magnetic bead-packing unit according to one of the previous claims, wherein the rotary body (100) is a compact disc-shaped rotating plate.

8. A microfluidic device comprising:
a rotary body controllably rotating and comprising a sample solution chamber (13), and a waste chamber (150) disposed farther from a rotation center of the rotary body than the sample solution chamber;
a microfluidic channel disposed on the rotary body and comprising an inlet (31) connected with the sample solution chamber (13) and an outlet (32) connected with the waste chamber (150) to provide a flow passage through which fluid flows from the inlet to the outlet due to centrifugal force, wherein the microfluidic channel comprises a curved portion in which the flow passage first extends away from the rotation center of the rotary body and then turns toward the rotation center of the rotary body; and
a magnet (40) disposed to correspond to the curved portion and applying a magnetic force to fluid flowing through the microfluidic channel.

9. The microfluidic device of claim 8, further comprising a bottleneck portion (136) in which a flow passage is reduced in size at a position close to the curved portion (135).

10. The microfluidic device of claim 9, wherein the bottleneck portion (136) is disposed at a rear end portion of the curved portion (135) along the low passage.

11. The microfluidic device according to one of claims 8 to 10, wherein the magnet (40) is fixed to the rotary body at a position adjacent to the curved portion (135).

12. The microfluidic device according to one of claims 8 to 11, wherein the magnet (40) is formed in the shape of a ring having a radius of rotation corresponding to the distance from the rotation center of the rotary body to the curved portion (135), and is disposed outside the rotary body.

13. The microfluidic device according to one of claims 8 to 12, wherein the curved portion (135) is U-shaped or V-shaped such that an outer portion of the curved portion faces a rim of the rotating body.

14. The microfluidic device according to one of claims 8 to 13, wherein the rotary body (100) is a compact disc-shaped rotating plate.

15. The microfluidic device according to one of claims 8 to 14, further comprising a valve (120) controlling a flow of fluid between the sample solution chamber and the inlet of the microfluidic channel.

16. The microfluidic device according to one of claims 8 to 15, wherein the diameter of magnetic bead (15) ranges from 10 to 50 µm.

17. The microfluidic device according to one of claims 8 to 15, wherein each of the magnetic beads (15) comprises a core (16) formed of a magnetic material and a shell (17) formed of a nonmagnetic material to surround the core.

18. The microfluidic device according to one of claims 8 to 15, wherein the shell is formed of a biologically inert polymer material and a surface of the shell is biologically activated.

19. The microfluidic device according to one of claims 8 to 15, wherein the shell (17) is formed of any one material selected from the group consisting of styrene, agarose, dextran, and polyethylene glycol (PEG).

20. The microfluidic device according to one of claims 8 to 15, wherein the surface of the shell (17) is modified such that predetermined biomolecules are specifically bound thereto.

21. The microfluidic device according to one of claims 8 to 15, wherein the surface of the shell (17) has at least one probe selected from the group consisting of an antibody, antigen, DNA, biotin, protein, amino group (NH₂-), and carboxyl group (COOH-).

22. The microfluidic device according to one of claims 8 to 15, wherein the surface of the shell (17) is formed of silica.

23. An immunoassay method using the microfluidic device of one of claims 8 to 22, the immunoassay method comprising:
preparing a sample solution mixed with magnetic beads (15) in the sample solution chamber; and
packing the magnetic beads (15) in the curved portion (135) by passing the sample solution mixed with the magnetic beads through the microfluidic channel (130,131,132,135,136) due to centrifugal force caused by rotation of the rotary body (100).
